# EUROPEAN PATENT SPECIFICATION

(11) **EP 3 456 705 B1**
(45) Date of publication and mention of the grant of the patent: **16.12.2020**
(21) Application number: 17795980.6
(22) Date of filing: 27.04.2017
(51) Int. Cl.: C07C 201/10, C07C 201/06, C07C 205/12, C07C 205/58

(54) **METHOD FOR PRODUCING NITROBENZENE COMPOUND**
VERFAHREN ZUR HERSTELLUNG EINER NITROBENZOLVERBINDUNG
PROCÉDÉ DE PRODUCTION D'UN COMPOSÉ DE NITROBENZÈNE

(30) Priority: 09.05.2016 JP 2016093792
(43) Date of publication of application: 20.03.2019
(73) Proprietor: Kumiai Chemical Industry Co., Ltd., Taito-ku Tokyo 110-8782 (JP)
(72) Inventor: NAKAMURA, Takayuki, Fuji-shi Shizuoka 421-3306 (JP); KATO, Akira, Fuji-shi Shizuoka 421-3306 (JP); ENOMOTO, Masaki, Fuji-shi Shizuoka 421-3306 (JP); TAFUJI, Jun, Fuji-shi Shizuoka 421-3306 (JP)
(74) Representative: Adam, Holger
(86) International application number: PCT/JP2017/016700
(87) International publication number: WO 2017/195619

(56) References cited:
- EP-A1- 2 754 651
- EP-A1- 3 015 451
- WO-A1-2013/005425
- WO-A1-2014/208296
- CN-A- 105 216 407
- JP-A- S 615 061
- JP-B2- 2 606 291
- FENG RONG-XIU ET AL: "Synthesis of 3-Chloro-2- Nitrotoluene", TRANSACTIONS OF TIANJIN UNIVERSITY, EDITORIAL BOARD OF TRANSACTIONS OF TIANJIN UNIVERSITY, TIANJIN, CN, vol. 8, no. 1, 1 March 2002 (2002-03-01), pages 40-42, XP008182596, ISSN: 1006-4982

## Description

### Technical Field

The present invention relates to a method for producing a nitrobenzene compound having chemical formula (1): 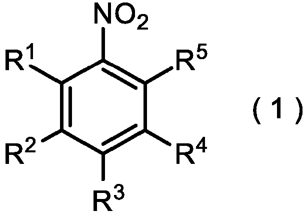 wherein R¹ represents a halogen atom, R², R³, and R⁴ may be the same or different from one another and each represent a hydrogen atom, a halogen atom, or an alkyl group, and R⁵ represents a halogen atom or an alkoxycarbonyl group.

### Background Art

Nitrobenzene compounds having chemical formula (1) are useful as intermediates to be used to produce various beneficial organic compounds. Particularly, 2,6-dichloronitrobenzene (see Patent Documents 1 and 2) and 2-chloro-6-alkoxycarbonylnitrobenzenes (see Patent Documents 3 and 4) are known as intermediates to be used to produce medicines, and agricultural chemicals, and the like.

As a method for producing a nitrobenzene compound having chemical formula (1), there is known a production method that involves diazotization of an aniline compound and subsequent nitration (see Patent Document 5 and Non-Patent Document 1). However, this method has some problems. One of the problems is that economic efficiency is low due to the use of a large amount of water as a solvent. Another problem is that a dinitro compound is formed as a by-product. Here, the dinitro compound refers to a dinitrobenzene compound. That is, the dinitro compound refers to a compound having two nitro groups on its benzene ring. Such dinitro compounds are generally known to be very dangerous. From an industrial point of view, formation of the dinitro compound is undesirable even in a small amount. That is, there has been desired a method capable of reducing the amount of a dinitro compound to be formed as a by-product.

Meanwhile, as another method for producing a nitrobenzene compound having chemical formula (1), there is known a method that involves oxidation of an aniline compound with hydrogen peroxide (see Patent Document 6). In this method, hydrogen peroxide, which should be carefully handled to keep the safety of industrial operations, is used. The method described in Patent Document 6 is superior to conventional art known before Patent Document 6, but still has a room for improvement in the use of hydrogen peroxide.

The present inventors have developed a method in which an aniline derivative is reacted in the presence of a metal nitrite and an acid and then a resulting product or a reaction mixture containing the resulting product is further reacted in the presence of a metal nitrite and a copper compound to produce a corresponding nitrobenzene derivative, wherein the total amount of the water to be used for the reactions is 1.2 to 2.2 L per 1 mol of the raw material aniline derivative (see Patent Document 7). This method not only is a production method suitable for industrial production because it can reduce the amount of a dangerous dinitro compound formed as a by-product to improve safety and uses no hydrogen peroxide, but also is a method excellent as an industrial production method with high economical efficiency because it can greatly reduce the amount of the metal nitrite to be used and also can reduce the amount of water to be used.

### Citation List

### Patent Document

Patent Document 1: JP 2005-533756 A
Patent Document 2: JP 2008-537953 A
Patent Document 3: WO 2005/081960 A2
Patent Document 4: US 5084086 A
Patent Document 5: JP 2606291 B2
Patent Document 6: WO 2013/005425 A1
Patent Document 7: WO 2014/208296 A1 (EP 3015451 A1)

### Non-Patent Document

Non-Patent Document 1: Transactions of Tianjin University, 2002, Vol. 8, No. 1, pp. 40-41

### Summary of the Invention

### Problems to be Solved by the Invention

It is an object of the present invention to provide a superior method having been more improved as an industrial method for producing a nitrobenzene compound having chemical formula (1).

Although the method disclosed in Patent Document 7 is a superior method as an industrial production method, but it is not necessarily satisfactory in terms of safety of operations, yield, etc., and an object of the present invention is to provide a method having been improved in such aspects.

### Means for Solving the Problems

Although it has been disclosed in Patent Document 7 that hydrochloric acid is preferred as an acid, suppression of thermal runaway during a reaction process is not sufficient even in the event that hydrochloric acid is used as an acid and it has been therefore desired to develop a safer method. The yield is on the order of 90% at most even when hydrochloric acid is used as an acid, and therefore a method better in this aspect has been desired.

Under these circumstances, the present inventors have further investigated a method for producing a nitrobenzene compound having chemical formula (1). As a result, the present inventors have unexpectedly found that by use of nitric acid as an acid, it not only is possible to better suppress thermal runaway during a reaction process to provide a safer industrial production method but also is possible to remarkably improve the yield. It is found that the above problem can be solved by use of not hydrochloric acid or sulfuric acid but nitric acid as an acid, that is, a method that involves reacting an aniline compound having chemical formula (2): 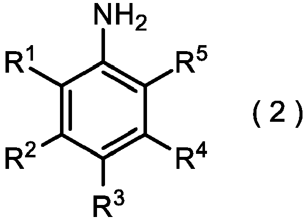 wherein, in the chemical formula (2), R¹ represents a halogen atom, R², R³, and R⁴ are the same or different from one another and each represent a hydrogen atom, a halogen atom, or an alkyl group, and R⁵ represents a halogen atom or an alkoxycarbonyl group, in the presence of a metal nitrite and nitric acid, and then reacting a resulting product or a reaction mixture containing the product in the presence of a metal nitrite and a copper compound, wherein the total amount of water used in the reactions is 1.2 to 2.2 L per 1 mol of the compound having chemical formula (2), and a nitrobenzene compound having chemical formula (1): 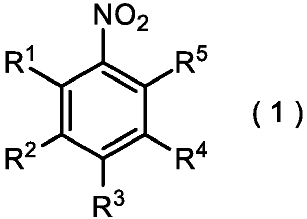 wherein, in the chemical formula (1), R¹, R², R³, R⁴, and R⁵ are as defined above, is thereby produced. The present inventors have completed the present invention based on this finding.

More specifically, the present invention provides the following.
[1] A method for producing a nitrobenzene compound having chemical formula (1): 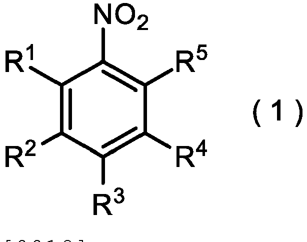 wherein, in the chemical formula (1), R¹ represents a halogen atom, R², R³, and R⁴ may be the same or different from one another and each represent a hydrogen atom, a halogen atom, or an alkyl group, R⁵ represents a halogen atom or an alkoxycarbonyl group,
   the method comprising the processes of:
   (i) reacting an aniline compound having chemical formula (2): 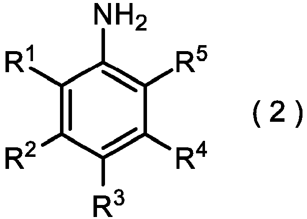 wherein, in the chemical formula (2), R¹, R², R³, R⁴, and R⁵ are as defined above, in the presence of a metal nitrite and nitric acid; and
   (ii) reacting a product of the process (i) or a reaction mixture containing the product (hereinafter referred to as a product of the process (i)) in the presence of a metal nitrite and a copper compound.
[2] The method according to the above [1], wherein the process (ii) is a process of performing the reaction in the presence of water and the total amount of water used in the process (ii) is 1.2 to 2.2 L per 1 mol of the compound having chemical formula (2).
[3] The method according to the above [2], wherein the total amount of water used in the process (ii) is 1.2 to 1.9 L per 1 mol of the compound having chemical formula (2).
[4] The method according to any one of the above [1] to [3], wherein the metal nitrite used in the process (i) or the process (ii) is an alkali metal nitrite or an alkaline earth metal nitrite.
[5] The method according to any one of the above [1] to [4], wherein the amount of the metal nitrite used in the process (i) is 1.0 to 10.0 mol per 1 mol of the compound having chemical formula (2).
[6] The method according to any one of the above [1] to [5], wherein the amount of the metal nitrite used in the process (i) is 1.0 to 3.0 mol per 1 mol of the compound having chemical formula (2).
[7] The method according to any one of the above [1] to [6], wherein the amount of the metal nitrite used in the process (ii) is 1.0 to 10.0 mol per 1 mol of the compound having chemical formula (2).
[8] The method according to any one of the above [1] to [7], wherein the amount of the metal nitrite used in the process (ii) is 1.0 to 3.0 mol per 1 mol of the compound having chemical formula (2).
[9] The method according to any one of the above [1] to [8], wherein the amount of the copper compound used in the process (ii) is 0.01 to 5.0 mol per 1 mol of the compound having chemical formula (2).
[10] The method according to any one of the above [1] to [9], wherein the amount of the copper compound used in the process (ii) is 0.01 to 0.5 mol per 1 mol of the compound having chemical formula (2).
[11] The method according to any one of the above [1] to [10], wherein the copper compound used in the process (ii) is copper oxide, a copper (I) salt, or a copper (II) salt.
[12] The method according to any one of the above [1] to [11], wherein the method further comprises the process of adjusting the pH of the product of the process (i) with a base.
[13] The method according to the above [12], wherein the base is a base selected from the group consisting of sodium hydroxide, potassium hydroxide, sodium carbonate, potassium carbonate, sodium hydrogen carbonate, and potassium hydrogen carbonate, or a mixture thereof.
[14] The method according to the above [12] or [13], wherein the base is sodium hydrogen carbonate.
[15] The method according to any one of the above [12] to [14], wherein the pH adjusted with the base is 0.5 to 3.0.
[16] The method according to any one of the above [12] to [14], wherein the pH adjusted with the base is 0.6 to 2.0.
[17] The method according to any one of the above [1] to [16], wherein the method further comprises the process in which the product of the process (i) or the product of the process (i) whose pH has been adjusted with a base is washed with an organic solvent.
[18] The method according to the above [17], wherein the organic solvent is an organic solvent selected from the group consisting of diisopropyl ether, dibutyl ether, cyclopentyl methyl ether (CPME), isobutyl methyl ketone (MIBK), ethyl acetate, butyl acetate, benzene, toluene, xylene, chlorobenzene, dichlorobenzene, and trichlorobenzene, or a mixture thereof.
[19] The method according to the above [17] or [18], wherein the organic solvent is toluene.
[20] The method according to any one of the above [1] to [11], wherein the process (ii) is performed in the presence of a base.
[21] The method according to the above [20], wherein the base is a base selected from the group consisting of sodium hydroxide, potassium hydroxide, sodium carbonate, potassium carbonate, sodium hydrogen carbonate, and potassium hydrogen carbonate, or a mixture thereof.
[22] The method according to the above [20] or [21], wherein the base is sodium hydrogen carbonate.
[23] The method according to any one of the above [1] to [22], wherein the process (ii) is performed in a aqueous solvent system containing an organic solvent.
[24] The method according to the above [23], wherein the organic solvent is an organic solvent selected from the group consisting of isobutyl methyl ketone (MIBK), ethyl acetate, butyl acetate, benzene, toluene, xylene, chlorobenzene, dichlorobenzene, and dichloromethane, or a mixture thereof.
[25] The method according to the above [23] or [24], wherein the organic solvent is toluene.
[26] The method according to any one of the above [1] to [25], wherein R², R³, and R⁴ are hydrogen atoms.
[27] The method according to any one of the above [1] to [26], wherein R¹ is a halogen atom and R⁵ is a (C1-C4)alkoxycarbonyl group.
[28] The method according to the above [27], wherein R¹ is a chlorine atom and R⁵ is a (C1-C4)alkoxycarbonyl group.
[29] The method according to the above [28], wherein R¹ is a chlorine atom and R⁵ is a methoxycarbonyl.
[30] The method according to any one of the above [1] to [26], wherein R¹ is a halogen atom and R⁵ is a halogen atom.
[31] The method according to the above [30], wherein R¹ is a chlorine atom and R⁵ is a chlorine atom.

### Effects of the Invention

According to the present invention, there is provided an industrially beneficial method for producing a nitrobenzene compound having chemical formula (1).

The method of the present invention is characterized in that nitric acid is used as the acid in the method disclosed in Patent Document 7 and has beneficial features, such as that it is a method using no hydrogen peroxide, that the amount of a dangerous dinitro compound formed as a by-product can be reduced and the safety is improved by adjusting the amount of water to be used, and that the amount of wastes can be reduced, and has beneficial effects that it can be performed by simple operations under mild conditions without using any specially designed reaction apparatus, which are disclosed in Patent Document 7.

Moreover, the method of the present invention has a particularly beneficial effect that it not only can suppress thermal runaway during the processes by using nitric acid as an acid but also is superior in the yield of the nitrobenzene compound having chemical formula (1).

Therefore, the method of the present invention provides the method disclosed in Patent Document 7 that has further been improved and not only is a method that is suitable for industrial production and allows safe and stable operation but also is a method capable of efficiently producing a nitrobenzene compound having chemical formula (1) with a high purity and yield. Moreover, the method of the present invention is friendly to the environment and is of high industrial utility value.

### Description of Embodiments

Hereinafter, the present invention will be described in detail.

Terms and symbols used in this description will be described below.

The halogen atom may be, for example, a fluorine atom, a chlorine atom, a bromine atom, or an iodine atom, preferably may be a fluorine atom or a chlorine atom, and more preferably may be a chlorine atom.

The term "Ca-Cb" means that the number of carbon atoms is from a to b. For example, the term "(C1-C4)" in the term "(C1-C4)alkyl group" means that the number of carbon atoms of the alkyl group is 1 to 4.

The alkyl group may be, for example, a (C1-C4)alkyl group. The (C1-C4)alkyl group may specifically be, for example, methyl, ethyl, propyl, isopropyl, butyl, sec-butyl, isobutyl, or tert-butyl, preferably may be methyl, ethyl, propyl, or isopropyl, more preferably may be methyl or ethyl, and even more preferably may be methyl.

The alkoxycarbonyl group may be, for example, a (C1-C4)alkoxycarbonyl group. The (C1-C4)alkoxycarbonyl group may specifically be, for example, methoxycarbonyl, ethoxycarbonyl, propoxycarbonyl, isopropoxycarbonyl, butoxycarbonyl, sec-butoxycarbonyl, isobutoxycarbonyl, or tert-butoxycarbonyl, preferably may be methoxycarbonyl, ethoxycarbonyl, propoxycarbonyl, isopropoxycarbonyl, more preferably may be methoxycarbonyl or ethoxycarbonyl, and even more preferably may be methoxycarbonyl.

### (Process (i))

First, process (i) will be described.

The process (i) is a process in which an aniline compound having chemical formula (2): 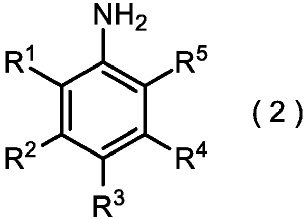 wherein R¹ represents a halogen atom, R², R³, and R⁴ are the same or different from one another and each represent a hydrogen atom, a halogen atom, or an alkyl group, and R⁵ represents a halogen atom or an alkoxycarbonyl group, is reacted in the presence of a metal nitrite and nitric acid

### (Raw Material: Aniline Compound Having Chemical Formula (2))

The aniline compound of the above chemical formula (2) is used as a raw material for the method according to the present invention. The aniline compound having chemical formula (2) is a known compound or a compound that can be produced from a known compound by a known method. Specific examples of the aniline compound having chemical formula (2) include, but are not limited to,
2,6-dichloroaniline,
2,6-dibromoaniline,
2,6-difluoroaniline,
2-chloro-6-fluoroaniline,
2-bromo-6-chloroaniline,
2-chloro-6-iodoaniline,
methyl 2-animo-3-chlorobenzoate,
ethyl 2-amino-3-chlorobenzoate,
propyl 2-amino-3-chlorobenzoate,
isopropyl 2-amino-3-chlorobenzoate,
butyl 2-amino-3-chlorobenzoate,
isobutyl 2-amino-3-chlorobenzoate,
sec-butyl 2-amino-3-chlorobenzoate,
tert-butyl 2-amino-3-chlorobenzoate,
methyl 2-amino-3-fluorobenzoate,
ethyl 2-amino-3-fluorobenzoate,
propyl 2-amino-3-fluorobenzoate,
isopropyl 2-amino-3-fluorobenzoate,
butyl 2-amino-3-fluorobenzoate,
isobutyl 2-amino-3-fluorobenzoate,
sec-butyl 2-amino-3-fluorobenzoate,
tert-butyl 2-amino-3-fluorobenzoate,
methyl 2-amino-3-bromobenzoate,
ethyl 2-amino-3-bromobenzoate,
propyl 2-amino-3-bromobenzoate,
isopropyl 2-amino-3-bromobenzoate,
methyl 2-amino-3-iodobenzoate, and
ethyl 2-amino-3-iodobenzoate. In addition, the aniline compound having chemical formula (2) may be a salt with an acid such as hydrochloric acid, sulfuric acid, or nitric acid.

### (Metal Nitrite Used in Process (i))

Examples of the metal nitrite that can be used in the process (i) include, but are not limited to, an alkali metal nitrite (e.g., lithium nitrite, sodium nitrite, or potassium nitrite) and an alkaline earth metal nitrite (e.g., magnesium nitrite, calcium nitrite, or barium nitrite). From the viewpoint of cost, availability, and reactivity, the metal nitrite is preferably an alkali metal nitrite, more preferably sodium nitrite or potassium nitrite, even more preferably sodium nitrite.

The metal nitrite to be used in the process (i) may be a salt different from, or alternatively may be a salt identical to the metal nitrite to be used in the process (ii), which will be described later. Namely, the metal nitrite to be used in the process (i) may be either the same as or different from the metal nitrite to be used in the process (ii).

The metal nitrite may be in any form as long as the reaction proceeds. The form of metal nitrite may be, for example, a solid consisted of solely metal nitrite(s), an aqueous solution of metal nitrite(s) of any concentration, or a solution of metal nitrite(s) solved by a solvent other than water. Further, the alkali metal nitrites may be used singly or in combination of two or more species of them at any ratio.

### (Amount of Metal Nitrite Used in Process (i))

The amount of the metal nitrite used is not particularly limited as long as the reaction proceeds.

From the viewpoint of yield, suppression of by-product formation, and economic efficiency, the amount of the metal nitrite used is, for example, usually in the range of 1.0 to 10.0 mol, preferably 1.0 to 5.0 mol, more preferably 1.0 to 3.0 mol, even more preferably 1.0 to 2.0 mol, particularly preferably 1.0 to 1.2 mol per 1 mol of the aniline compound having chemical formula (2).

### (Acid Used in Process (i))

The method of the present invention is characterized in that nitric acid is used as an acid to be used in the process (i). It is preferable to use an aqueous nitric acid solution as the nitric acid. The concentration of nitric acid in the aqueous nitric acid solution is not particularly limited, but there is used an aqueous solution having a concentration of nitric acid of 30% to 90% is usually used, and preferably, an aqueous solution having a concentration of nitric acid of 68% to about 70%, at which an azeotrope is to be formed.

The nitric acid in the present invention does not exclude its use in combination with another acid, e.g., an inorganic acid such as hydrochloric acid and sulfuric acid, but it is usually preferable to use nitric acid alone.

### (Amount of Nitric Acid Used in Process (i))

The amount of the nitric acid used is not particularly limited as long as the reaction proceeds. From the viewpoint of yield, suppression of by-product formation, and economic efficiency, the amount of the acid used is, for example, usually in the range of 1 to 10 mol, preferably 1 to 5 mol, more preferably 1 to 3 mol per 1 mol of the aniline compound having chemical formula (2).

### (Solvent Used in Process (i))

From the viewpoint of allowing the reaction to smoothly proceed, the reaction of the process (i) is preferably performed in the presence of solvents. The solvent used in the process (i) is not particularly limited as long as the reaction of the process (i) proceeds and the reaction of a process (ii) is not adversely affected. From the viewpoint of cost and handleability, the solvent used in the process (i) is particularly preferably water.

However, solvents other than water, which will be described later, are not excluded as long as a desired reaction proceeds. For example, water and a solvent other than water may be used as long as a desired reaction proceeds. Examples of the solvent, other than water, that can be used in the process (i) include, but are not limited to: alcohols (e.g., methanol, ethanol, 2-propanol, and butanol); ethers (e.g., tetrahydrofuran (THF), 1,4-dioxane, diisopropyl ether, dibutyl ether, cyclopentyl methyl ether (CPME), methyl-tert-butyl ether, 1,2-dimethoxyethane (DME), diglyme, and triglyme); nitriles (e.g., acetonitrile); amides (e.g., N,N-dimethylformamide (DMF), N,N-dimethylacetamide (DMAC), and N-methylpyrrolidone (NMP)); alkyl ureas (e.g., N,N'-dimethylimidazolidinone (DMI)); sulfoxides (e.g., dimethylsulfoxide (DMSO)); sulfones (e.g., sulfolane); ketones (e.g., acetone and isobutyl methyl ketone (MIBK)); carboxylates (e.g., ethyl acetate and butyl acetate); carboxylic acids (e.g., acetic acid); aromatic hydrocarbon derivatives (e.g., benzene, toluene, xylene, chlorobenzene, dichlorobenzene, trichlorobenzene, and nitrobenzene); and halogenated aliphatic hydrocarbons (e.g., dichloromethane).

From the viewpoint of cost, handleability, reactivity, and yield, examples of the solvent, other than water, that can be used in the process (i) preferably include alcohols, ethers, nitriles, amides, sulfoxides, ketones, aromatic hydrocarbon derivatives, and halogenated aliphatic hydrocarbons, more preferably include ketones.

Specific examples of the solvent, other than water, that can be used in the process (i) preferably include methanol, ethanol, tetrahydrofuran (THF), dibutyl ether, acetonitrile, N,N-dimethylformamide (DMF), N,N-dimethylacetamide (DMAC), N-methylpyrrolidone (NMP), dimethylsulfoxide (DMSO), isobutyl methyl ketone (MIBK), toluene, xylene, chlorobenzene, dichlorobenzene, trichlorobenzene, and dichloromethane, more preferably include isobutyl methyl ketone (MIBK).

### (Amount of Solvent Used in Process (i))

The amount of the solvent used in the process (i) is not particularly limited as long as a reaction system can be sufficiently stirred. From the viewpoint of reactivity, suppression of by-product formation, and economic efficiency, the total amount of water used in the process (i) is, for example, usually in the range of 0.1 to 1.5 L (liters), preferably 0.3 to 1.3 L, more preferably 0.4 to 1.2 L per 1 mol of the aniline compound having chemical formula (2). Here, the total amount of water used in the process (i) refers to the amount of all water present in the reaction system at the time when the reaction of the process (i) is finished. Therefore, when the nitric acid or the metal nitrite added is in the form of an aqueous solution, not only the amount of water added as a solvent but also the amount of water in the aqueous solution is added. For example, the total amount of water used in the process (i) includes the amount of water in the aqueous solution of the nitric acid used in the process (i) and the amount of water in the aqueous solution of the metal nitrite used in the process (i). Further, from the same point of view, the amount of the solvent other than water is, for example, usually in the range of 0 (zero) to 5 L (liters), preferably 0 to 1 L per 1 mol of the aniline compound having chemical formula (2). It is to be noted that, when water and a solvent other than water are used in combination, the ratio between water and the solvent other than water is not particularly limited as long as the reaction proceeds. When two or more solvents other than water are used, the ratio between/among the two or more solvents other than water is not particularly limited as long as the reaction proceeds. Meanwhile, it is particularly preferred that water is used singly as a solvent or water is used in combination with an organic solvent immiscible with water.

### (Reaction Temperature of Process (i))

The reaction temperature of the process (i) is not particularly limited. From the viewpoint of yield, suppression of by-product formation, and economic efficiency, the reaction temperature is, for example, usually in the range of -30°C (minus 30°C) to 50°C, preferably -20°C to 25°C, more preferably -10°C to 10°C, and even more preferably -5°C to 5°C.

### (Reaction Time of Process (i))

The reaction time of the process (i) is not particularly limited. From the viewpoint of yield, suppression of by-product formation, and economic efficiency, the reaction time is, for example, usually in the range of 0.1 hours to 48 hours, preferably 0.1 hours to 24 hours, more preferably 0.1 hours to 12 hours, and even more preferably 0.1 hours to 3 hours.

### (Product of Process (i))

The product of the process (i) is a diazonium salt compound corresponding to the aniline compound having chemical formula (2) used as a raw material. The diazonium salt compound is a compound generally well-known to those skilled in the art.

In the present invention, the term "product of the process (i)" includes not only a purified or isolated reaction product of the process (i) but also an unrefined crude product and a mixture containing a reaction product of the process (i). Such a mixture may be, for example, a reaction mixture itself of the process (i) or only a layer, e.g., a water layer, of the reaction mixture containing a reaction product of the process (i). Such a mixture may further be refined, if necessary.

When only the water layer portion of the reaction mixture of the process (i) is used for the subsequent process (ii), it is preferable to wash the reaction mixture of the process (i) with an organic solvent that is not miscible with water. Examples of the organic solvent that is not miscible with water include, but are not limited to, ethers (e.g., diisopropyl ether, dibutyl ether, cyclopentyl methyl ether (CPME), and methyl tert-butyl ether), ketones (e.g., isobutyl methyl ketone (MIBK)), carboxylates (e.g., ethyl acetate and butyl acetate), aromatic hydrocarbon derivatives (e.g., benzene, toluene, xylene, chlorobenzene, dichlorobenzene, trichlorobenzene, and nitrobenzene), and halogenated aliphatic hydrocarbons (e.g., dichloromethane).

From the viewpoint of cost, handleability, reactivity, and yield, preferred examples of the organic solvent that is not miscible with water include ethers, ketones, carboxylates, and aromatic hydrocarbon derivatives, and more preferably include aromatic hydrocarbon derivatives.

Specific preferable examples of the organic solvent that is not miscible with water include diisopropyl ether, dibutyl ether, cyclopentyl methyl ether (CPME), isobutyl methyl ketone (MIBK), ethyl acetate, butyl acetate, benzene, toluene, xylene, chlorobenzene, dichlorobenzene, and trichlorobenzene, and more preferably include toluene.

The amount of the organic solvent to be used is not particularly limited as long as it is sufficient for the washing, but it is preferably 50 mL to 300 mL, more preferably about 100 mL to about 200 mL per 1 mol of the aniline compound having chemical formula (2).

When washing the reaction mixture of the process (i) with an organic solvent, water required in the process (ii) may be added at the same time. That is, the organic solvent that is not miscible with water and the water may be added simultaneously and perform the washing.

The washing with an organic solvent can be performed by adding the organic solvent or a mixture of the organic solvent and water to the reaction mixture of the process (i) or a reaction mixture resulting from pH adjustment of the reaction mixture of the process (i) by the addition of a base, subsequently distributing an organic layer and a water layer, and then separating the water layer.

### (Use of Base)

In the method of the present invention, a base may be added to a mixture after the completion of the reaction of the process (i) or before the start of the reaction of the process (ii). In other words, the pH of the reaction system of the process (ii) may be adjusted by adding a base, if necessary. The addition of a base, that is, the adjustment of pH may or may not be performed as long as the reaction of the process (ii) smoothly proceeds. Further, the addition of a base may be performed anytime and anywhere (in any reaction container or the like) as long as the reaction of the process (ii) smoothly proceeds.

The range of the pH adjusted by the addition of the base is not particularly limited as long as the reaction can proceed smoothly, but it is preferably a range of pH 0.5 to 3.0, more preferably a range of pH 0.6 to 2.0, for example.

### (Base)

Examples of the base include, but are not limited to: an alkali metal hydroxide (e.g., lithium hydroxide, sodium hydroxide, or potassium hydroxide); an alkaline earth metal hydroxide (e.g., magnesium hydroxide, calcium hydroxide, or barium hydroxide); an alkali metal carbonate (e.g., lithium carbonate, sodium carbonate, or potassium carbonate); an alkaline earth metal carbonate (e.g., magnesium carbonate, calcium carbonate, or barium carbonate); an alkali metal hydrogen carbonate (e.g., lithium hydrogen carbonate, sodium hydrogen carbonate, or potassium hydrogen carbonate); an alkaline earth metal hydrogen carbonate (e.g., magnesium hydrogen carbonate, calcium hydrogen carbonate, or barium hydrogen carbonate); a phosphate (e.g., sodium phosphate, potassium phosphate, or calcium phosphate); a hydrogen phosphate (e.g., sodium hydrogen phosphate, potassium hydrogen phosphate, or calcium hydrogen phosphate); an alkali metal carboxylate (e.g., sodium formate, potassium formate, lithium acetate, sodium acetate, or potassium acetate); an alkaline earth metal carboxylate (e.g., magnesium acetate or calcium acetate); and ammonia.

From the viewpoint of cost, handleability, reactivity, and yield, preferable examples of the base include an alkali metal hydroxide, an alkali metal carbonate, and an alkali metal hydrogen carbonate, more preferably include an alkali metal hydrogen carbonate.

Specific examples of the base preferably include sodium hydroxide, potassium hydroxide, sodium carbonate, potassium carbonate, sodium hydrogen carbonate, and potassium hydrogen carbonate, more preferably include sodium hydrogen carbonate or potassium hydrogen carbonate, and even more preferably include sodium hydrogen carbonate.

The base may be in any form as long as the reaction proceeds. The form of the base may be, for example, solid or liquid consisting of solely a base, an aqueous solution of a base of any concentration, or a solution of a base solved by a solvent other than water. Further, the bases may be used singly or in combination of two or more species of them at any ratio.

### (Amount of Base Used)

The amount of the base used can be determined after consideration by those skilled in the art, if necessary. The amount of the base used is not particularly limited as long as the reaction proceeds. From the viewpoint of yield, suppression of by-product formation, and economic efficiency, the amount of the base used is, for example, usually in the range of 0 (zero) to 5 mol, preferably 0 to 1 mol, more preferably 0 to 0.6 mol per 1 mol of the aniline compound having chemical formula (2).

### (Process (ii))

Hereinafter, the process (ii) will be described.

The process (ii) is a process in which the product of the process (i) is reacted in the presence of a metal nitrite and a copper compound to produce a compound having chemical formula (1): 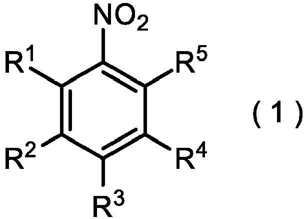 wherein R¹, R², R³, R⁴, and R⁵ are as defined above.

### (Metal Nitrite Used in Process (ii))

Examples of the metal nitrite that can be used in the process (ii) include, but are not limited to: an alkali metal nitrite (e.g., lithium nitrite, sodium nitrite, or potassium nitrite); and an alkaline earth metal nitrite (e.g., magnesium nitrite, calcium nitrite, or barium nitrite). From the viewpoint of cost, availability, and reactivity, the metal nitrite is preferably an alkali metal nitrite, more preferably sodium nitrite or potassium nitrite, even more preferably sodium nitrite.

The metal nitrite may be in any form as long as the reaction proceeds. The form of metal nitrite may be, for example, a solid consisted of solely metal nitrite(s), an aqueous solution of metal nitrite(s) of any concentration, or a solution of metal nitrite(s) solved by a solvent other than water. Further, the alkali metal nitrites may be used singly or in combination of two or more species of them at any ratio.

### (Amount of Metal Nitrite Used in Process (ii))

The amount of the metal nitrite used is not particularly limited as long as the reaction proceeds. From the viewpoint of yield, suppression of by-product formation, and economic efficiency, the amount of the metal nitrite used is, for example, usually in the range of 1.0 to 10.0 mol, preferably 1.0 to 7.0 mol, more preferably 1.0 to 5.0 mol, even more preferably 1.0 to 3.0 mol per 1 mol of the aniline compound having chemical formula (2).

### (Copper Compound Used in Process (ii))

In the process (ii), a copper compound is preferably used as a catalyst. As a copper compound that can be used in the process (ii), one generally known as a catalyst for Sandmeyer reaction can be mentioned. Further, a catalyst used in a method proposed by Hantzsch et al. can be mentioned. Examples of the copper compound include copper oxide, a copper (I) salt, a copper (II) salt, and a double salt including copper (I) and copper (II), and copper powder. Examples of the copper oxide include copper (I) oxide and copper (II) oxide. Examples of the copper (I) salt include copper (I) sulfate, copper (I) sulfite, copper (I) carbonate, copper (I) chloride, copper (I) bromide, and copper (I) cyanide. Examples of the copper (II) salt include copper (II) sulfate, copper (II) sulfite, copper (II) carbonate, copper (II) chloride, copper (II) bromide, and copper (II) cyanide. Examples of the double salt including copper (I) and copper (II) include copper (I) copper (II) sulfite (cupro-cupri sulfite) that is a catalyst used in a method proposed by Hantzsch et al..

Specific examples of the copper compound that can be used in the process (ii) preferably include, but are not limited to, copper (I) oxide, copper (I) sulfate, copper (I) sulfite, copper (I) copper (II) sulfite (cupro-cupri sulfite), copper (I) carbonate, and copper powder, more preferably include copper (I) oxide, copper (I) sulfate, copper(I) sulfite, copper (I) copper (II) sulfite (cupro-cupri sulfite), and copper powder, and even more preferably include copper (I) oxide and copper (I) copper (II) sulfite (cupro-cupri sulfite).

As described above, the copper compound may be either a simple salt or a double salt. Further, the copper compound may be either an anhydride or a hydrate. The copper compound may be in any form as long as the reaction proceeds. The form of copper compound may be, for example, a solid consisting of solely copper compound(s), an aqueous solution of copper compound of any concentration, or a solution of copper compound(s) solved by a solvent other than water. Further, the copper compounds may be used singly or in combination of two or more species of them at any ratio.

### (Amount of Copper Compound Used in Process (ii))

The amount of the copper compound used is not particularly limited as long as the reaction proceeds. From the viewpoint of yield, suppression of by-product formation, economic efficiency, and safety, the amount of the copper compound used is, for example, usually in the range of 0.01 to 5.0 mol, preferably 0.01 to 1.0 mol, more preferably 0.01 to 0.5 mol per 1 mol of the aniline compound having chemical formula (2).

### (Solvent Used in Process (ii))

From the viewpoint of allowing the reaction to smoothly proceed, the reaction of the process (ii) is preferably performed in the presence of a solvent. The solvent used in the process (ii) is not particularly limited as long as the reaction of the process (ii) proceeds. Examples of the solvent that can be used in the process (ii) include, but are not limited to, water; alcohols (e.g., methanol, ethanol, 2-propanol, and butanol); ethers (e.g., tetrahydrofuran (THF), 1,4-dioxane, diisopropyl ether, dibutyl ether, cyclopentyl methyl ether (CPME), methyl-tert-butyl ether, 1,2-dimethoxyethane (DME), diglyme, and triglyme); nitriles (e.g., acetonitrile); amides (e.g., N,N-dimethylformamide (DMF), N,N-dimethylacetamide (DMAC), N-methylpyrrolidone (NMP); alkyl ureas (e.g., N,N'-dimethylimidazolidinone (DMI)); sulfoxides (e.g., dimethylsulfoxide (DMSO)); sulfones (e.g., sulfolane); ketones (e. g., acetone and isobutyl methyl ketone (MIBK)); carboxylates (e.g., ethyl acetate and butyl acetate); carboxylic acids (e.g., acetic acid); aromatic hydrocarbon derivatives (e.g., benzene, toluene, xylene, chlorobenzene, dichlorobenzene, trichlorobenzene, and nitrobenzene); halogenated aliphatic hydrocarbons (e.g., dichloromethane); and mixed solvents composed of combinations of two or more species of these solvents at any ratio.

From the viewpoint of cost, handleability, reactivity, and yield, it is preferable to perform the reaction of the process (ii) in the presence of water. Preferable examples of the solvent used in the process (ii) include water alone, a combination of water and a ketone, a combination of water and a carboxylate, a combination of water and an aromatic hydrocarbon derivative, and a combination of water and a halogenated aliphatic hydrocarbon, more preferably include water alone, a combination of water and a ketone, and a combination of water and an aromatic hydrocarbon derivative, and even more preferably include a combination of water and an aromatic hydrocarbon derivative.

Specific examples of the solvent used in the process (ii) preferably include water alone, a combination of water and isobutyl methyl ketone (MIBK), a combination of water and ethyl acetate, a combination of water and butyl acetate, a combination of water and toluene, a combination of water and xylene, a combination of water and chlorobenzene, a combination of water and dichlorobenzene, and a combination of water and dichloromethane, more preferably include water alone, a combination of water and isobutyl methyl ketone (MIBK), a combination of water and toluene, a combination of water and xylene, a combination of water and chlorobenzene, and a combination of water and dichlorobenzene, even more preferably include a combination of water and toluene, a combination of water and xylene, a combination of water and chlorobenzene, and a combination of water and dichlorobenzene, and particularly preferably include a combination of water and toluene.

### (Amount of Solvent Used in Process (ii))

The amount of the solvent used in the process (ii) is not particularly limited as long as the reaction system can be sufficiently stirred. In the case of performing the reaction of the process (ii) in the presence of water, from the viewpoint of reactivity, suppression of by-product formation, and economic efficiency, the total amount of water used in the process (ii) is, for example, preferably in the range of 1.2 to 2.2 L (liters), more preferably 1.2 to 1.9 L per 1 mol of the aniline compound having chemical formula (2). Here, the total amount of water used in the process (ii) refers to the amount of all water present in the reaction system at the time when the reaction of the process (ii) is finished. Therefore, when the acid or the metal nitrite added is in the form of an aqueous solution, not only the amount of water added in the process (ii) but also the amount of water in the aqueous solution is added. Further, when the reaction mixture of the process (i) is directly used, the amount of water in the reaction mixture is also added. For example, the total amount of water used in the process (ii) includes the amount of water in an aqueous solution of the metal nitrite used in the process (ii) and the amount of water in an aqueous solution of the base used in the process (ii), and further includes the total amount of water used in the process (i) when the reaction mixture of the process (i) is directly used. From the same point of view, the amount of the solvent other than water is, for example, usually in the range of 0 (zero) to 5 L (liters), preferably 0.1 to 1 L, more preferably 0.2 to 0.9 L per 1 mol of the aniline compound having chemical formula (2). It is to be noted that, when water and a solvent other than water are used in combination, the ratio between water and the solvent other than water is not particularly limited as long as the reaction proceeds. When two or more solvents other than water are used, the ratio between/among the two or more solvents other than water is not particularly limited as long as the reaction proceeds.

### (Reaction Temperature of Process (ii))

The reaction temperature of the process (ii) is not particularly limited. From the viewpoint of yield, suppression of by-product formation, and economic efficiency, the reaction temperature is, for example, usually in the range of -30°C (minus 30°C) to 70°C, preferably -20°C to 50°C, more preferably -10°C to 35°C, and even more preferably -5°C to 10°C.

### (Reaction Time of Process (ii))

The reaction time of the process (ii) is not particularly limited. From the viewpoint of yield, suppression of by-product formation, and economic efficiency, the reaction time is, for example, usually in the range of 0.1 hours to 48 hours, preferably 0.1 hours to 24 hours, more preferably 0.5 hours to 12 hours, and even more preferably 0.5 hours to 1 hour.

### (Product of Process (ii) : Nitrobenzene Compound having chemical formula (1))

Specific examples of the nitrobenzene compound having chemical formula (1) obtained in the process (ii) include, but are not limited to:
2,6-dichloronitrobenzene,
2,6-dibromonitrobenzene,
2,6-difluoronitrobenzene,
2-chloro-6-fluoronitrobenzene,
2-bromo-6-chloronitrobenzene,
2-chloro-6-iodonitrobenzene,
methyl 3-chloro-2-nitrobenzoate,
ethyl 3-chloro-2-nitrobenzoate,
propyl 3-chloro-2-nitrobenzoate,
isopropyl 3-chloro-2-nitrobenzoate,
butyl 3-chloro-2-nitrobenzoate,
isobutyl 3-chloro-2-nitrobenzoate,
sec-butyl 3-chloro-2-nitrobenzoate,
tert-butyl 3-chloro-2-nitrobenzoate,
methyl 3-fluoro-2-nitrobenzoate,
ethyl 3-fluoro-2-nitrobenzoate,
propyl 3-fluoro-2-nitrobenzoate,
isopropyl 3-fluoro-2-nitrobenzoate,
butyl 3-fluoro-2-nitrobenzoate,
isobutyl 3-fluoro-2-nitrobenzoate,
sec-butyl 3-fluoro-2-nitrobenzoate,
tert-butyl 3-fluoro-2-nitrobenzoate,
methyl 3-bromo-2-nitrobenzoate,
ethyl 3-bromo-2-nitrobenzoate,
propyl 3-bromo-2-nitrobenzoate,
isopropyl 3-bromo-2-nitrobenzoate,
methyl 3-iodo-2-nitrobenzoate, and
ethyl 3-iodo-2-nitrobenzoate.

From the reaction mixture of the process (ii), a desired compound can be obtained by separating and purifying the desired nitrobenzene compound having chemical formula (1). Examples of a means for the separation and purification include ordinary means such as separation of insolubles by filtration, an extraction method, a distillation method, a recrystallization method, and chromatography. It is also possible to know the amount of the desired compound in the reaction mixture by high-performance liquid chromatography (HPLC) or gas chromatography (GC).

### (Yield in Process (i) and Process (ii))

The overall yield in the process (i) and the process (ii) in the method of the present invention can be adjusted to 90% or more by using nitric acid as an acid. More specifically, it can also be brought to 95% or more. Such a drastic improvement in yield is expected to be derived not only from an improvement in conversion ratio but also from an improvement in selectivity.

The overall yield in the process (i) and the process (ii) can be calculated as a ratio of the number of moles of the obtained nitrobenzene compound having chemical formula (1) to the number of moles of the aniline compound having chemical formula (2) used as a raw material. That is, the yield is represented by the following formula: Yield % = 100 × {(number of moles of obtained nitrobenzene compound having chemical formula (1)) / (number of moles of aniline compound having chemical formula (2) as raw material)}

### (Safety of Diazonium Salt Formed in Process (i))

The safety of the diazonium salts formed in the process (i) has been investigated. That is, the exotherm onset temperature (°C), the time required until thermal runaway occurs when the residence temperature (T_{R}) is kept (TMR (hr) at T_{R}), and the temperature at which the time required until thermal runaway occurs is 24 hours (ADT₂₄ (°C)) with diazonium salts formed according to the method of the present invention using nitric acid as an acid (see Examples 2 and 3 disclosed below) and diazonium salts formed according to a method involving use of hydrochloric acid as an acid (see Comparative Examples 1 and 8 disclosed below) were calculated from values measured by a differential scanning calorimetry and an accelerated rate calorimetry or the measured values. The residence temperature (T_{R}) in the TMR measurement was set with reference to the temperatures in practical operations, namely, the residence temperature in Example 2 and Comparative Example 1 was set to 5°C and the residence temperature in Example 3 and Comparative Example 8 was set to 0°C.

Next, description is made by taking the measurements in Example 2 and Comparative Example 1 as examples. The exotherm onset temperature (°C) was 137.25°C in Example 2, in which nitric acid was used, whereas it was 114.51°C in Comparative Example 1, in which hydrochloric acid was used. The reaction temperature of the process (ii) is usually no more than 5°C, which is sufficient, and is about 30°C at most, and therefore, the difference from the exotherm onset temperature is conceived large enough. However, it has been revealed that the method of the present invention using nitric acid is a method which involves a higher exotherm onset temperature and therefore is safer.

Moreover, TMR (hr) at T_{R} was 2385.6 hours in Example 2, in which nitric acid was used according to the present invention, whereas it was 255.3 hours in Comparative Example 1, in which hydrochloric acid was used. Considering the fact that 5°C is high enough as the reaction temperature of the process (ii) of the present invention and about 0.5 hours is long enough as the reaction time, a time 4000 times or longer is expected to be required until thermal runaway occurs in Example 2, in which nitric acid was used according to the present invention. On the other hand, thermal runaway could occur even at a time about 500 times longer in Comparative Example 1, in which hydrochloric acid was used. Also from the viewpoint of such a time required before thermal runaway occurs, it was confirmed that the method of the present invention was far safer.

Moreover, ADT₂₄ (°C) was 63.2°C in Example 2, in which nitric acid was used according to the present invention, whereas it was 38.8°C in Comparative Example 1, in which hydrochloric acid was used. In the case that the reaction temperature of the process (ii) is 5°C, thermal runaway could occur after 24 hours when the temperature has risen by 58.2°C from 5°C (the temperature has become 12.6 times) in Example 2, in which nitric acid was used according to the present invention, whereas thermal runaway could occur after 24 hours when the temperature has risen by only 33.8°C from 5°C (the temperature has become 7.8 times) in Comparative Example 1, in which hydrochloric acid was used. Also from the viewpoint of such temperature elevation, it was confirmed that Example 2, in which nitric acid was used, was safer about 2 times Comparative Example 1, in which hydrochloric acid was used.

These facts have shown that the method involving use of nitric acid according to the present invention exhibits not only improvement in yield but also significant improvement in safety of operation as compared with the method involving use of hydrochloric acid as disclosed in Patent Document 7. Use of nitric acid is expected to increase generation of dinitro compounds as by-products and reduce the safety of a reaction, but contrary to such expectations, the method involving use of nitric acid according to the present invention is safer than the method involving use of hydrochloric acid.

### Examples

Hereinafter, the producing method according to the present invention will be specifically described with reference to Examples, but is not intended that the present invention is limited thereto.

In the following Examples, room temperature usually refers to a range of 10°C to 35°C.

In the present specification, the following instruments were used for measuring the physical properties of the examples and comparative examples.

¹H nuclear magnetic resonance spectrometer (¹H-NMR): Varian Mercury-300, internal reference: tetramethylsilane (TMS)

(Gas Chromatography (GC) Analysis Method); GC-2010 (manufactured by Shimadzu Corporation), detection method: FID

The following documents can be referred to for the GC analysis method, as desired.
(a): "Shin-Jikkenkagaku Koza 9, Bunseki Kagaku II (A New Course in Experimental Chemistry Course 9, Analytical Chemistry II)", pp. 60 to 86 (1977), edited by The Chemical Society of Japan, published by Shingo Iizumi, Maruzen Company, Limited (for example, page 66 of this document can be referred to with respect to liquids for a stationary phase to be applicable for a column).
(b): "Jikkenkagaku Koza 20-1, Bunseki Kagaku (A Course in Experimental Chemistry 20-1, Analytical Chemistry)", 5th edition, pp. 121 to 129 (2007), edited by The Chemical Society of Japan, published by Seishiro Murata, Maruzen Company, Limited (for example, pages 124 to 125 of this document can be referred to with respect to the specific usage of hollow capillary separation columns).

### (High-Performance Liquid Chromatography (HPLC) Analysis Method); LC20AD (manufactured by Shimadzu Corporation)

The following documents can be referred to for the HPLC analysis method, as desired.
(a): "Shin-Jikkenkagaku Koza 9, Bunseki Kagaku II (A New Course in Experimental Chemistry 9, Analytical Chemistry II)", pp. 86 to 112 (1977), edited by The Chemical Society of Japan, published by Shingo Iizumi, Maruzen Company, Limited (for example, pages 93 to 96 of this document can be referred to with respect to a filler-mobile phase combination to be applicable for a column).
(b): "Jikkenkagaku Koza 20-1, Bunseki Kagaku (A Course in Experimental Chemistry 20-1, Analytical Chemistry)", 5th edition, pp. 130 to 151 (2007), edited by The Chemical Society of Japan, published by Seishiro Murata, Maruzen Company, Limited (for example, pages 135 to 137 of this document can be referred to with respect to the specific method and conditions for reverse phase chromatography analysis).

### (Method for Measuring pH)

The pH was measured by a glass electrode-type hydrogen-ion concentration indicator. As the glass electrode-type hydrogen-ion concentration indicator, for example, Model HM-20P manufactured by DKK-TOA CORPORATION can be used.

### (Differential Scanning Calorimetry)

Differential scanning calorimetry was carried out within a temperature range of 40 to 400°C at a heating rate of 10°C/min using an instrument: DSC-60 (manufactured by Shimadzu Corporation). The following documents can be referred to for the differential scanning calorimetry, as desired.
(a): "Dai-Yon Han, Jikkenkagaku Koza 4, Netsu, Atsuryoku (A Course in Experimental Chemistry 4, Heat, Pressure)", 4th Edition, pp. 57 to 93 (1992), edited by The Chemical Society of Japan, published by Kumao Ebihara, Maruzen Company, Limited.
(b): "Dai-Go Han, Jikkenkagaku Koza 6, Ondo, Netsu, Atsuryoku (A Course in Experimental Chemistry 6, Temperature, Heat, Pressure", 5th edition, pp. 203 to 205 (2005), edited by The Chemical Society of Japan, published by Seishiro Murata, Maruzen Company, Limited.

### (Accelerated Rate Calorimetry)

In accelerated rate calorimetry, a thermal behavior in an adiabatic state was measured within a temperature range of 40 to 400°C using an instrument: New ARC (registered trademark) (manufactured by TIAX LLC). ADT₂₄ is a temperature at which the time required until thermal runaway occurs is 24 hours, and the time required until thermal runaway occurs is referred to as TMR.

### Example 1

### Production of 2,6-dichloronitrobenzene using nitric acid

A 200-mL four-necked flask equipped with a stirrer, a reflux condenser, a thermometer, and a dropping funnel was charged with 16.2 g (0.10 mol) of 2,6-dichloroaniline, 81.7 mL of water, and 21 g (0.23 mol) of 69% nitric acid, and the resulting mixture was heated to 60°C and dissolved. The mixture was cooled to 37°C while being stirred, and after stirring at that temperature for 30 minutes, the mixture was further cooled to 0°C. Then, 19.0 g (0.11 mol) of a 40% aqueous sodium nitrite solution was dropped thereinto at 0 to 5°C over 25 minutes and then the resulting reaction mixture was stirred at 0 to 5°C for 30 minutes. Then, 36 mL of a 5% aqueous sodium hydrogen carbonate solution was dropped to adjust the pH of the reaction mixture to 0.6 to 1.3, and then 17.5 mL of toluene and 30 g of water were added. The mixture obtained above was distributed to a toluene layer and a water layer, and then the water layer was separated.

A 300-mL four-necked flask equipped with a stirrer, a reflux condenser, a thermometer, and a dropping funnel was charged with 2.15 g (0.015 mol) of copper (I) oxide, 34.9 mL of toluene, and 19.0 g (0.11 mol) of a 40% aqueous sodium nitrite solution, and the water layer of the reaction mixture obtained above was dropped thereinto at 0 to 5°C over 1 hour. The resulting reaction mixture was stirred at 0 to 4°C for 30 minutes, and then a filter aid was added and precipitates were removed by filtration. The resulting toluene layer was washed with 33.6 mL (0.04 mol) of a 10% aqueous sodium hydrogen carbonate solution to obtain 2,6-dichloronitrobenzene in the form of a toluene solution. The obtained toluene solution was analyzed by a GC internal reference method. As a result, the yield of 2,6-dichloronitrobenzene was 94.2%.

### Example 2

### Production of 2,6-dichloronitrobenzene using nitric acid

A 500-mL four-necked separable flask equipped with a stirrer, a reflux condenser, a thermometer, and a dropping funnel was charged with 32.4 g (0.20 mol) of 2,6-dichloroaniline, 190 mL of water, and 42.0 g (0.46 mol) of 69% nitric acid, and the resulting mixture was heated to 60°C and dissolved. The mixture was cooled to 37°C while being stirred, and after stirring at that temperature for 30 minutes, the mixture was further cooled to 0°C. Then, 38.0 g (0.22 mol) of a 40% aqueous sodium nitrite solution was dropped thereinto at -3 (minus 3) to 5°C over 35 minutes and then the resulting reaction mixture was stirred at -3 to 5°C for 30 minutes. Then, 72.2 mL of a 5% aqueous sodium hydrogen carbonate solution was dropped to adjust the pH of the reaction mixture to 0.8 to 2.0, and then 30.4 mL of toluene was added. The mixture obtained above was distributed to a toluene layer and a water layer, and then the water layer was separated. The resulting water layer was subjected to differential scanning calorimetry and accelerated rate calorimetry.

A 500-mL four-necked flask equipped with a stirrer, a reflux condenser, a thermometer, and a dropping funnel was charged with 1.43 g (0.01 mol) of copper (I) oxide, 69.9 mL of toluene, and 38.0 g (0.22 mol) of a 40% aqueous sodium nitrite solution, and the water layer of the reaction mixture obtained above was dropped thereinto at -3 to 5°C over 75 minutes. The reaction mixture was stirred at -3 to 5°C for 30 minutes, then 38.8 g (0.04 mol) of 10% sulfamic acid and 4 g (0.04 mol) of 35% hydrochloric acid were added and precipitates were removed by filtration. The filtrate was separated into a water layer and a toluene layer, the water layer was further extracted with 35.0 mL of toluene. The resulting toluene layers were combined and washed with 67.2 mL (0.08 mol) of a 10% aqueous sodium hydrogen carbonate solution to obtain 2,6-dichloronitrobenzene in the form of a toluene solution. The obtained toluene solution was analyzed by a GC internal reference method. As a result, the yield of 2,6-dichloronitrobenzene was 95.5%.

¹H-NMR (300MHz, CDCl₃) δ(ppm): 7.47-7.36 (m, 3H)

Differential scanning calorimetry: exotherm onset temperature: 137.25°C, exotherm amount: 551.92 J/g

Accelerated rate calorimetry: ADT₂₄: 63.2°C, TMR at T_{R}: 2385.6 hours

### Comparative Example 1

### Production of 2,6-dichloronitrobenzene using hydrochloric acid

A 200-mL four-necked flask equipped with a stirrer, a reflux condenser, a thermometer, and a dropping funnel was charged with 16.2 g (0.10 mol) of 2,6-dichloroaniline, 39.6 mL of water, and 24.0 g (0.23 mol) of 35% hydrochloric acid, and the resulting mixture was heated to 60°C and dissolved. The mixture was cooled to 37°C while being stirred, and after stirring at that temperature for 30 minutes, the mixture was further cooled to 0°C. Then, 19.0 g (0.11 mol) of a 40% aqueous sodium nitrite solution was dropped thereinto at 0 to 4°C over 20 minutes and then the resulting reaction mixture was stirred at 0 to 4°C for 30 minutes. Then, 36 mL of a 5% aqueous sodium hydrogen carbonate solution was dropped to adjust the pH of the reaction mixture to 0.6 to 1.3, and then 17.5 mL of toluene was added. The mixture obtained above was distributed to a toluene layer and a water layer, and then the water layer was separated. The resulting water layer was subjected to differential scanning calorimetry and accelerated rate calorimetry.

A 300-mL four-necked flask equipped with a stirrer, a reflux condenser, a thermometer, and a dropping funnel was charged with 1.08 g (0.0075 mol) of copper (I) oxide, 32.9 mL of water, 34.9 mL of toluene, and 19.0 g (0.11 mol) of a 40% aqueous sodium nitrite solution, and the water layer of the reaction mixture obtained above was dropped thereinto at 0 to 4°C over 75 minutes. The resulting reaction mixture was stirred at 0 to 4°C for 30 minutes, and then a filter aid was added and precipitates were removed by filtration. The filtrate was separated into a water layer and a toluene layer, and the resulting toluene layer was washed with 33.6 mL (0.04 mol) of a 10% aqueous sodium hydrogen carbonate solution to obtain 2,6-dichloronitrobenzene in the form of a toluene solution. The obtained toluene solution was analyzed by a GC internal reference method. As a result, the yield of 2,6-dichloronitrobenzene was 90.6%.

Differential scanning calorimetry: exotherm onset temperature: 114.51°C, exotherm amount: 503.39 J/g

Accelerated rate calorimetry: ADT₂₄: 38.8°C, TMR at T_{R}: 255.3 hours

In Comparative Example 1, in which hydrochloric acid was used, the reaction conditions were improved, but the yield was able to be improved to no more than about 90%. Moreover, thermal indices including the exotherm onset temperature (°C), ADT₂₄, and TMR at T_{R} were apparently inferior to those in the case of using nitric acid, which revealed that the improvement in safety is limited.

### Comparative Example 2

### Production of 2,6-dichloronitrobenzene using hydrochloric acid

A 2000-mL four-necked flask equipped with a stirrer, a reflux condenser, a thermometer, and a dropping funnel was charged with 162.0 g (1.00 mol) of 2,6-dichloroaniline, 396.4 mL of water, and 239.6 g (2.30 mol) of 35% hydrochloric acid. The mixture was heated to 60°C and dissolved. The mixture was cooled to -5°C (minus 5°C) under stirring, and then, 199.7 g (1.10 mol) of a 38% aqueous sodium nitrite solution was dropped thereinto at -5 to 0°C. Then, 174.7 mL of toluene was added and 420 mL of a saturated aqueous sodium hydrogen carbonate solution was dropped at -5 to 0°C to adjust the pH to 3. The mixture was distributed to toluene and water, and then the water layer was separated.

A 3000-mL four-necked flask equipped with a stirrer, a reflux condenser, a thermometer, and a dropping funnel was charged with 48.4 g (0.34 mol) of copper (I) oxide, 349.5 mL of toluene, and 544.7 g (3.00 mol) of a 38% aqueous sodium nitrite solution, and the water layer obtained above was dropped thereinto at 0 to 5°C over 2 hours. The mixture was stirred at 0 to 5°C for 30 minutes and then copper was removed by filtration. The filtrate was distributed into toluene and water and then the toluene layer was separated. The resulting toluene layer was washed with 336.0 mL (0.40 mol) of a saturated aqueous sodium hydrogen carbonate solution to obtain 2,6-dichloronitrobenzene in the form of a toluene solution. The obtained toluene solution was analyzed by a GC internal reference method. As a result, the yield of 2,6-dichloronitrobenzene was 85%.

Comparative Example 2 is an example disclosed as Example 5 in Patent Document 7. The yield was no more than 85%.

### Comparative Example 3

### Production of 2,6-dichloronitrobenzene using sulfuric acid

A 200-mL four-necked flask equipped with a stirrer, a reflux condenser, a thermometer, and a dropping funnel was charged with 16.2 g (0.10 mol) of 2,6-dichloroaniline, 39.6 mL of water, and 39.1 g (0.23 mol) of 57.6% sulfuric acid, and the resulting mixture was heated to 60°C and dissolved. The mixture was cooled to 37°C while being stirred, and after stirring at that temperature for 30 minutes, the mixture was further cooled to 0°C. Then, 19.0 g (0.11 mol) of a 40% aqueous sodium nitrite solution was dropped thereinto at 0 to 4°C over 40 minutes and then the resulting reaction mixture was stirred at 0 to 4°C for 10 minutes. Then, 17.5 mL of toluene was added and 36 mL of a 5% aqueous sodium hydrogen carbonate solution was dropped. Further, 1.93 g (0.02 mol) of powdered sodium hydrogen carbonate was added and the pH of the reaction mixture was adjusted to 0.21. The mixture obtained above was distributed to a toluene layer and a water layer, and then the water layer was separated.

A 300-mL four-necked flask equipped with a stirrer, a reflux condenser, a thermometer, and a dropping funnel was charged with 2.15 g (0.015 mol) of copper (I) oxide, 34.9 mL of toluene, and 19.0 g (0.11 mol) of a 40% aqueous sodium nitrite solution, and the water layer of the reaction mixture obtained above was dropped thereinto at 0 to 4°C over 2 hours. The resulting reaction mixture was stirred at 0 to 4°C for 30 minutes, and then a filter aid was added and precipitates were removed by filtration. The filtrate was separated into a water layer and a toluene layer, and the resulting toluene layer was washed with 33.6 mL (0.04 mol) of a 10% aqueous sodium hydrogen carbonate solution to obtain 2,6-dichloronitrobenzene in the form of a toluene solution. The obtained toluene solution was analyzed by a GC internal reference method. As a result, the yield of 2,6-dichloronitrobenzene was 28.7%.

Comparative Example 3 is an example in which sulfuric acid was used as an acid. The yield was no more than 28.7%.

### Comparative Example 4

### Production of 2,6-dichloronitrobenzene using sodium hydrogen sulfate

A 200-mL four-necked flask equipped with a stirrer, a reflux condenser, a thermometer, and a dropping funnel was charged with 16.2 g (0.10 mol) of 2,6-dichloroaniline, 39.6 mL of water, and 76.2 g (0.23 mol) of 41.7% aqueous sodium hydrogen sulfate solution, and the resulting mixture was heated to 60°C and dissolved. The mixture was cooled to 37°C while being stirred, and after stirring at that temperature for 30 minutes, the mixture was further cooled to 0°C. Then, 19.0 g (0.11 mol) of a 40% aqueous sodium nitrite solution was dropped thereinto at 0 to 4°C over 25 minutes and then the resulting reaction mixture was stirred at 0 to 4°C for 30 minutes. Then, 17.5 mL of toluene was added and 36 mL of a 5% aqueous sodium hydrogen carbonate solution was dropped to adjust the pH of the reaction mixture to 1.11. The mixture obtained above was distributed to a toluene layer and a water layer, and then the water layer was separated.

A 300-mL four-necked flask equipped with a stirrer, a reflux condenser, a thermometer, and a dropping funnel was charged with 2.15 g (0.015 mol) of copper (I) oxide, 34.9 mL of toluene, and 19.0 g (0.11 mol) of a 40% aqueous sodium nitrite solution, and the water layer of the reaction mixture obtained above was dropped thereinto at 0 to 4°C over 70 minutes. The resulting reaction mixture was stirred at 0 to 4°C for 30 minutes, and then a filter aid was added and precipitates were removed by filtration. The filtrate was separated into a water layer and a toluene layer, and the resulting toluene layer was washed with 33.6 mL (0.04 mol) of a 10% aqueous sodium hydrogen carbonate solution to obtain 2,6-dichloronitrobenzene in the form of a toluene solution. The obtained toluene solution was analyzed by a GC internal reference method. As a result, the yield of 2,6-dichloronitrobenzene was 52.3%.

Comparative Example 4 is an example in which sodium hydrogen sulfate was used as an acid. The yield was no more than 52.3%.

### Comparative Example 5

### Production of 2,6-dichloronitrobenzene using tetrafluoroboric acid

A 100-mL four-necked flask equipped with a stirrer, a reflux condenser, a thermometer, and a dropping funnel was charged with 8.1 g (0.05 mol) of 2,6-dichloroaniline, 15.9 mL of water, and 24.0 g (0.115 mol) of a 42% aqueous tetrafluoroboric acid solution, and the resulting mixture was heated to 60°C and dissolved. The mixture was cooled to 37°C while being stirred, and after stirring at that temperature for 30 minutes, the mixture was further cooled to 0°C. Then, 9.5 g (0.055 mol) of a 40% aqueous sodium nitrite solution was dropped thereinto at 0 to 4°C over 20 minutes and then the resulting reaction mixture was stirred at 0 to 4°C for 45 minutes. Then, 8.7 mL of toluene was added and 18 mL of a 5% aqueous sodium hydrogen carbonate solution was dropped to adjust the pH of the reaction mixture to 0.4, and then 20 mL of water was added.

A 300-mL four-necked flask equipped with a stirrer, a reflux condenser, a thermometer, and a dropping funnel was charged with 1.1 g (0.0077 mol) of copper (I) oxide, 17.5 mL of toluene, and 9.5 g (0.055 mol) of a 40% aqueous sodium nitrite solution, and the reaction mixture obtained above was dropped thereinto at 0 to 4°C over 1 hour. The solid remaining in the dropping funnel at the time of dropping was poured into the flask by further adding 40g of water. The resulting reaction mixture was stirred at 0 to 4°C for 30 minutes, and then a filter aid was added and precipitates were removed by filtration. The filtrate was separated into a water layer and a toluene layer, and the resulting toluene layer was washed with 16.8 mL (0.02 mol) of a 10% aqueous sodium hydrogen carbonate solution to obtain 2,6-dichloronitrobenzene in the form of a toluene solution. The obtained toluene solution was analyzed by a GC internal reference method. As a result, the yield of 2,6-dichloronitrobenzene was 40%.

Comparative Example 5 is an example in which tetrafluoroboric acid was used as an acid. The yield was no more than 40%.

### Comparative Example 6

### Production of 2,6-dichloronitrobenzene using acetic acid

A 200-mL four-necked flask equipped with a stirrer, a reflux condenser, a thermometer, and a dropping funnel was charged with 16.2 g (0.10 mol) of 2,6-dichloroaniline, 85.5 mL of water, and 13.9 g (0.23 mol) of acetic acid, and the resulting mixture was heated to 60°C and dissolved. The mixture was cooled to 37°C while being stirred, and after stirring at that temperature for 30 minutes, the mixture was further cooled to 0°C. Then, 19.0 g (0.11 mol) of a 40% aqueous sodium nitrite solution was dropped thereinto at 0 to 4°C over 25 minutes and then the resulting reaction mixture was stirred at 0 to 4°C for 30 minutes. Then, 32.7 mL of toluene and 36.7 mL of water were added to the reaction mixture.

A 300-mL four-necked flask equipped with a stirrer, a reflux condenser, a thermometer, and a dropping funnel was charged with 2.15 g (0.015 mol) of copper (I) oxide, 34.9 mL of toluene, and 19.0 g (0.11 mol) of a 40% aqueous sodium nitrite solution, and the reaction mixture obtained above was dropped thereinto at 0 to 4°C over 75 minutes. The resulting reaction mixture was stirred at 0 to 4°C for 30 minutes, and then a filter aid was added and precipitates were removed by filtration. The filtrate was separated into a water layer and a toluene layer, and the resulting toluene layer was washed with 33.6 mL (0.04 mol) of a 10% aqueous sodium hydrogen carbonate solution to obtain 2,6-dichloronitrobenzene in the form of a toluene solution. The obtained toluene solution was analyzed by a GC internal reference method. As a result, the yield of 2,6-dichloronitrobenzene was 2.8%.

Comparative Example 6 is an example in which acetic acid was used as an acid. The yield was no more than 2.8%.

### Example 3

### Production of methyl 3-chloro-2-nitrobenzoate using nitric acid

A 300-mL four-necked flask equipped with a stirrer, a reflux condenser, a thermometer, and a dropping funnel was charged with 106.1 g (0.20 mol) of a 35% solution of methyl 2-amino-3-chlorobenzoate in isobutyl methyl ketone (MIBK) and 96.8 mL of water. Then, 42 g (0.46 mol) of 69% nitric acid was dropped over 30 minutes while stirring the mixture, and the mixture was cooled to -5°C to 0°C with further stirring. Then, 39.9 g (0.22 mol) of a 38% aqueous sodium nitrite solution was dropped thereinto at -5°C to 0°C over 1 hour and then the resulting reaction mixture was stirred at -5°C to 0°C for 1 hour. The mixture obtained above was distributed to a MIBK layer and a water layer, and then the water layer was separated. The resulting water layer was subjected to differential scanning calorimetry and accelerated rate calorimetry.

A 1000-mL four-necked flask equipped with a stirrer, a reflux condenser, a thermometer, and a dropping funnel was charged with 9.7 g (0.068 mol) of copper (I) oxide, 22.5 mL of water, 168 mL of a 10% aqueous sodium hydrogen carbonate solution, 174 mL of toluene, and 72.6 g (0.40 mol) of a 38% aqueous sodium nitrite solution, and the water layer of the reaction mixture obtained above was dropped thereinto at 0 to 10°C over 2 hours. The resulting reaction mixture was stirred at 0 to 10°C for 1 hour, and then a filter aid was added and precipitates were removed by filtration. The obtained toluene layer was washed with 40 mL of water to obtain methyl 3-chloro-2-nitrobenzoate in the form of a toluene solution. The obtained toluene solution was analyzed by an HPLC absolute calibration curve method. As a result, the yield of methyl 3-chloro-2-nitrobenzoate was 95.2%.

¹H-NMR (300MHz, CDCl₃) δ (ppm): 7.56-8.01 (m, 3H), 3.92 (s, 3H)

Differential scanning calorimetry: exotherm onset temperature: 133.63°C, exotherm amount: 120.48 J/g

Accelerated rate calorimetry: ADT₂₄: 27.0°C, TMR at T_{R}: 78.6 hours

### Example 4

### Production of methyl 3-chloro-2-nitrobenzoate using nitric acid

A 300-mL four-necked flask equipped with a stirrer, a reflux condenser, a thermometer, and a dropping funnel was charged with 37.12 g (0.20 mol) of methyl 2-amino-3-chlorobenzoate and 96.8 mL of water, and the resulting mixture was heated to 55°C and dissolved. Then, 42 g (0.46 mol) of 69% nitric acid was dropped over 30 minutes while stirring the mixture, and the mixture was cooled to -5°C to 0°C with further stirring. Then, 39.9 g (0.22 mol) of a 38% aqueous sodium nitrite solution was dropped thereinto at -5°C to 0°C over 45 minutes and then the resulting reaction mixture was stirred at -5°C to 0°C for 1 hour. Then, 17.4 mL of toluene was added, and the obtained mixture was distributed to a toluene layer and a water layer, and then the water layer was separated.

A 1000-mL four-necked flask equipped with a stirrer, a reflux condenser, a thermometer, and a dropping funnel was charged with 9.7 g (0.068 mol) of copper (I) oxide, 22.5 mL of water, 168 mL of a 10% aqueous sodium hydrogen carbonate solution, 174 mL of toluene, and 72.6 g (0.40 mol) of a 38% aqueous sodium nitrite solution, and the water layer of the reaction mixture obtained above was dropped thereinto at 0 to 10°C over 2 hours. The resulting reaction mixture was stirred at 0 to 10°C for 1 hour, and then a filter aid was added and precipitates were removed by filtration. The obtained toluene layer was washed with 40 mL of water to obtain methyl 3-chloro-2-nitrobenzoate in the form of a toluene solution. The obtained toluene solution was analyzed by an HPLC absolute calibration curve method. As a result, the yield of methyl 3-chloro-2-nitrobenzoate was 94.9%.

### Comparative Example 7

### Production of methyl 3-chloro-2-nitrobenzoate using hydrochloric acid

A 100-mL four-necked flask equipped with a stirrer, a reflux condenser, a thermometer, and a dropping funnel was charged with 9.28 g (0.050 mol) of methyl 2-amino-3-chlorobenzoate, 24 mL of water, and 10.95 g (0.105 mol) of 35% hydrochloric acid. While the mixture was stirred at - 5°C (minus 5°C), and then, 9.35 g (0.0515 mol) of a 38% aqueous sodium nitrite solution was dropped thereinto. Then, the mixture was stirred at -5 to 0°C for 2 hours to obtain a reaction mixture.

A 200-mL four-necked flask equipped with a stirrer, a reflux condenser, a thermometer, and a dropping funnel was charged with 2.42 g (0.0169 mol) of copper (I) oxide, 25 mL of toluene, 27.24 g (0.150 mol) of a 38% aqueous sodium nitrite solution, and 21 g of water, and the reaction mixture obtained above was dropped thereinto at 25°C over 2 hours. To the resulting reaction mixture was added 25 mL of toluene, and the resulting mixture was stirred at 30°C for 10 minutes and then copper was removed by filtration. The filtrate was distributed into toluene and water and then the toluene layer was separated. The obtained toluene layer was washed with 25 mL of water to obtain methyl 3-chloro-2-nitrobenzoate in the form of a toluene solution. The obtained toluene solution was analyzed by an HPLC absolute calibration curve method. As a result, the yield of methyl 3-chloro-2-nitrobenzoate was 88%. At this time, the content of impurity methyl 2,3-dinitrobenzoate was 0.6%.

Comparative Example 7 is an example disclosed as Example 1 in Patent Document 7, in which methyl 2-amino-3-chlorobenzoate was used as a raw material. The yield of the nitro compound was no more than 88%.

### Comparative Example 8

### Production of methyl 3-chloro-2-nitrobenzoate using hydrochloric acid

A 300-mL four-necked flask equipped with a stirrer, a reflux condenser, a thermometer, and a dropping funnel was charged with 106.1 g (0.20 mol) of a 35% solution of methyl 2-amino-3-chlorobenzoate in isobutyl methyl ketone (MIBK) and 78.6 mL of water. Then, 47.90 g (0.460 mol) of 35% hydrochloric acid was dropped over 30 minutes while stirring the mixture, and the mixture was cooled to -5°C to 0°C with further stirring. Then, 39.9 g (0.22 mol) of a 38% aqueous sodium nitrite solution was dropped thereinto at -5°C to 0°C over 1 hour and then the resulting reaction mixture was stirred at -5°C to 0°C for 1 hour. The mixture obtained above was distributed to a MIBK layer and a water layer, and then the water layer was separated. The resulting water layer was subjected to differential scanning calorimetry and accelerated rate calorimetry.

A 1000-mL four-necked flask equipped with a stirrer, a reflux condenser, a thermometer, and a dropping funnel was charged with 9.7 g (0.068 mol) of copper (I) oxide, 22.5 mL of water, 168 mL of a 10% aqueous sodium hydrogen carbonate solution, 174 mL of toluene, and 72.6 g (0.40 mol) of a 38% aqueous sodium nitrite solution, and the water layer of the reaction mixture obtained above was dropped thereinto at 0 to 10°C over 2 hours. The resulting reaction mixture was stirred at 0 to 10°C for 1 hour, and then precipitates were removed by filtration. The obtained toluene layer was washed with 40 mL of water to obtain methyl 3-chloro-2-nitrobenzoate in the form of a toluene solution. The obtained toluene solution was analyzed by an HPLC absolute calibration curve method. As a result, the yield of methyl 3-chloro-2-nitrobenzoate was 74.3%.

Differential scanning calorimetry: exotherm onset temperature: 72.22°C, exotherm amount: 171.65 J/g

Accelerated rate calorimetry: ADT₂₄: 2.5°C, TMR at T_{R}: 25.6 hours

In Comparative Example 8, in which hydrochloric acid was used, the yield was about 74%, which was inferior to Example 3, in which nitric acid was used. Moreover, the results regarding thermal indices including exotherm onset temperature (°C), ADT₂₄, and TMR at T_{R} were apparently inferior to those in Example 3 in which nitric acid was used, and this showed that improvement in safety was limited.

### Industrial Applicability

According to the method of the present invention using nitric acid as an acid, there is provided a safer method for producing a nitrobenzene compound having chemical formula (1) with a higher yield.

The method according to the present invention is economical and friendly to the environment and has a great deal of potential in industry. There is provided a simple, safe and efficient method for producing a nitrobenzene compound having chemical formula (1) that serves as a raw material of chemical products such as various medicines and agricultural chemicals, and therefore the method has industrial applicability.

## Claims

1. A method for producing a nitrobenzene compound having chemical formula (1): 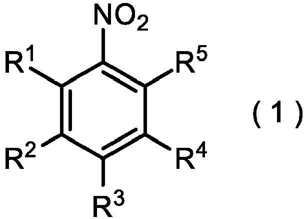 wherein, in the chemical formula (1), R¹ represents a halogen atom, R², R³, and R⁴ may be the same or different from one another and each represent a hydrogen atom, a halogen atom, or an alkyl group, and R⁵ represents a halogen atom or an alkoxycarbonyl group,
the method comprising the processes of:
(i) reacting an aniline compound having chemical formula (2): 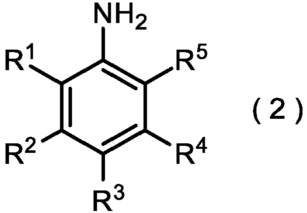 wherein, in the chemical formula (2), R¹, R², R³, R⁴, and R⁵ are as defined above, in the presence of a metal nitrite and nitric acid; and
(ii) reacting a product of the process (i) or a reaction mixture of the process (i) in the presence of a metal nitrite and a copper compound.

2. The method according to claim 1, wherein the process (ii) is a process of performing the reaction in the presence of water and a total amount of water used in the process (ii) is 1.2 to 2.2 L per 1 mol of the compound having chemical formula (2).

3. The method according to claim 2, wherein the total amount of water used in the process (ii) is 1.2 to 1.9 L per 1 mol of the compound having chemical formula (2).

4. The method according to any one of claims 1 to 3, wherein R², R³, and R⁴ are hydrogen atoms.

5. The method according to any one of claims 1 to 4, wherein R¹ is a halogen atom and R⁵ is a halogen atom.

6. The method according to any one of claims 1 to 5, wherein R¹ is a chlorine atom and R⁵ is a chlorine atom.

7. The method according to any one of claims 1 to 4, wherein R¹ is a halogen atom and R⁵ is a (C1-C4)alkoxycarbonyl group.

8. The method according to any one of claims 1 to 4,
wherein R¹ is a chlorine atom and R⁵ is a (C1-C4)alkoxycarbonyl group.

9. The method according to claim 8, wherein R¹ is a chlorine atom and R⁵ is methoxycarbonyl.

## Patentansprüche

1. Verfahren zur Herstellung einer Nitrobenzolverbindung mit der chemischen Formel (1): 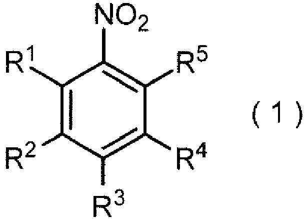 wobei, in der chemischen Formel (1), R¹ ein Halogenatom darstellt, R², R³ und R⁴ gleich oder voneinander verschieden sein können und jeweils ein Wasserstoffatom, ein Halogenatom oder eine Alkylgruppe darstellen, und R⁵ ein Halogenatom oder eine Alkoxycarbonylgruppe darstellt,
wobei das Verfahren die Verfahrensschritte umfasst:
(i) Umsetzung einer Anilinverbindung mit der chemischen Formel (2): 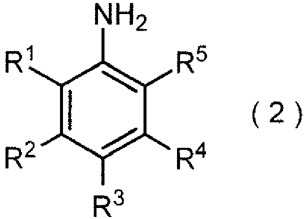 wobei, in der chemischen Formel (2), R¹, R², R³, R⁴ und R⁵ wie vorstehend definiert sind, in Gegenwart eines Metallnitrids und Salpetersäure; und
(ii) Umsetzung eines Produkts des Verfahrensschrittes (i) oder eines Reaktionsgemisches des Verfahrensschrittes (i) in Gegenwart eines Metallnitrids und einer Kupferverbindung.

2. Verfahren gemäß Anspruch 1, wobei der Verfahrensschritt (ii) ein Verfahren der Durchführung der Umsetzung in Gegenwart von Wasser ist und eine Gesamtmenge des verwendeten Wassers in dem Verfahrensschritt (ii) gleich 1, 2 bis 2,2 L pro 1 mol der Verbindung mit der chemischen Formel (2) ist.

3. Verfahren gemäß Anspruch 2, wobei die Gesamtmenge des verwendeten Wassers in dem Verfahrensschritt (ii) gleich 1,2 bis 1,9 L pro 1 mol der Verbindung mit der chemischen Formel (2) ist.

4. Verfahren gemäß einem der Ansprüche 1 bis 3, wobei R², R³ und R⁴ Wasserstoffatome sind.

5. Verfahren gemäß einem der Ansprüche 1 bis 4, wobei R¹ ein Halogenatom ist und R⁵ ein Halogenatom ist.

6. Verfahren gemäß einem der Ansprüche 1 bis 5, wobei R¹ ein Chloratom ist und R⁵ ein Chloratom ist.

7. Verfahren gemäß einem der Ansprüche 1 bis 4, wobei R¹ ein Halogenatom ist und R⁵ eine (C1-C4)Alkoxycarbonylgruppe ist.

8. Verfahren gemäß einem der Ansprüche 1 bis 4, wobei R¹ ein Chloratom ist und R⁵ eine (C1-C4)Alkoxycarbonylgruppe ist.

9. Verfahren gemäß Anspruch 8, wobei R¹ ein Chloratom ist und R⁵ Methoxycarbonyl ist.

## Revendications

1. Procédé de production d'un composé de nitrobenzène présentant la formule chimique (1) : 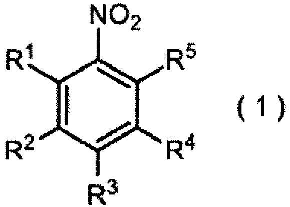 dans lequel, dans la formule chimique (1), R¹ représente un atome d'halogène, R², R³ et R⁴ peuvent être identiques ou différents les uns des autres et représentent chacun un atome d'hydrogène, un atome d'halogène, ou un groupe alkyle, et R⁵ représente un atome d'halogène ou un groupe alcoxycarbonyle,
le procédé comprenant les processus consistant à :
(i) faire réagir un composé d'aniline présentant la formule chimique (2) : 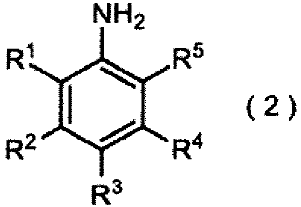 dans lequel, dans la formule chimique (2), R¹, R², R³, R⁴, et R⁵ sont tels que définis ci-dessus, en présence d'un nitrite métallique et d'acide nitrique ; et
(ii) faire réagir un produit du processus (i) ou un mélange réactionnel du processus (i) en présence d'un nitrite métallique et d'un composé de cuivre.

2. Procédé selon la revendication 1, dans lequel le processus (ii) est un processus consistant à effectuer la réaction en présence d'eau et une quantité totale d'eau utilisée dans le processus (ii) est de 1,2 à 2,2 L pour 1 mole du composé présentant la formule chimique (2).

3. Procédé selon la revendication 2, dans lequel la quantité totale d'eau utilisée dans le processus (ii) est de 1,2 à 1,9 L pour 1 mole du composé présentant la formule chimique (2).

4. Procédé selon l'une quelconque des revendications 1 à 3, dans lequel R², R³ et R⁴ sont des atomes d'hydrogène.

5. Procédé selon l'une quelconque des revendications 1 à 4, dans lequel R¹ est un atome d'halogène et R⁵ est un atome d'halogène.

6. Procédé selon l'une quelconque des revendications 1 à 5, dans lequel R¹ est un atome de chlore et R⁵ est un atome de chlore.

7. Procédé selon l'une quelconque des revendications 1 à 4, dans lequel R¹ est un atome d'halogène et R⁵ est un groupe (C1-C4)alcoxycarbonyle.

8. Procédé selon l'une quelconque des revendications 1 à 4,
dans lequel R¹ est un atome de chlore et R⁵ est un groupe (C1-C4)alcoxycarbonyle.

9. Procédé selon la revendication 8, dans lequel R¹ est un atome de chlore et R⁵ est un méthoxycarbonyle.
